(19) Europäisches Patentamt
European Patent Office
Office européen des brevets

(11) **EP 4 767 946 A1**

(12) **EUROPEAN PATENT APPLICATION**
published in accordance with Art. 153(4) EPC

(43) Date of publication:
**01.07.2026 Bulletin 2026/27**

(21) Application number: **24856060.9**

(22) Date of filing: **14.02.2024**

(51) International Patent Classification (IPC):
**A61B 6/03** (2006.01)     **A61B 1/00** (2006.01)

(52) Cooperative Patent Classification (CPC):
**A61B 1/00; A61B 6/03**

(86) International application number:
**PCT/JP2024/004959**

(87) International publication number:
**WO 2025/041364 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.08.2023 JP 2023134343**

(71) Applicant: **Boston Medical Sciences, Inc.
Tokyo 103-0023 (JP)**

(72) Inventor: **OKAMOTO Masaki
Tokyo 103-0023 (JP)**

(74) Representative: **Mathys & Squire
32 London Bridge Street
The Shard
London SE1 9SG (GB)**

(54) **ACQUISITION METHOD FOR VIRTUALLY CLEANED INTESTINAL TRACT IMAGE**

(57)     [Problem]
To provide a method or program for obtaining a virtual large intestine cleansing image that can remove residual matter from large intestine imaging data captured without the use of laxatives.
[Solution]
The method includes:
an image input step, in which a captured image representing a part of a patient's large intestine is input; and a virtual large intestine cleansing step, in which a virtual large intestine cleansing image representing a part of the patient's large intestine with residual matter removed from the captured image is obtained. The invention also provides a program for causing a computer to execute the above method for obtaining virtual large intestine cleansing images.

[Fig. 2]

**Description**

**TECHNICAL FIELD**

The present method relates to a method for acquiring a virtually cleansed intestinal tract image. More specifically, the method relates to a technique for obtaining an image of the large intestine after virtually cleansing the intestinal tract by performing image processing to remove residues in an image, based on a CT image or the like of the patient's large intestine.

**BACKGROUND ART**

Japanese Patent No. 7304594 describes a system that uses deep learning to determine whether tissues included in medical images are normal or abnormal. On the other hand, when diagnosing diseases of the large intestine, such as the presence of polyps or colorectal cancer, it may be difficult to make a determination based solely on medical images due to residues (e.g., feces) in the intestinal tract. Because residues are present in the intestines, it is not easy to accurately grasp the condition of the intestinal tract. Therefore, it is common to administer a laxative and, after removing the intestinal residues, examine the intestinal environment using an endoscope.

**PRIOR ART DOCUMENTS**

**Patent Literatures**

[Patent Literature 1] Japanese Patent No. 7304594

**SUMMARY OF INVENTION**

**Technical Problem**

One of the objectives of the present invention is to provide a method for acquiring a virtually cleansed intestinal tract image capable of removing residues from a captured image of the large intestine taken without using a laxative.
Another objective of the present invention is to provide a method for acquiring a virtually cleansed intestinal tract image that can prevent situations that interfere with the diagnosis of large intestine-related diseases.
Another objective of the present invention is to provide a method for detecting large intestine-related diseases, particularly polyps and colorectal cancer, from a captured image of the large intestine.
Another objective of the present invention is to provide a generative AI model for generating virtually cleansed intestinal tract images, and a generative AI model for detecting large intestine-related diseases.

**Means for solving the technical problem**

The present invention aims to achieve any one of the above objectives.
The present invention is based on the insight that, by performing image processing to remove residues from a captured image of a portion of a patient's large intestine, it is possible to obtain a virtually cleansed intestinal tract image, which is an image of the intestinal tract that has been virtually cleansed. Furthermore, the present invention is based on the insight that, by using machine learning, it is possible to provide a method for acquiring a virtually cleansed intestinal tract image that can suppress situations interfering with the diagnosis of large intestine-related diseases, without administering a laxative to the patient.
This specification describes a method for acquiring a virtually cleansed intestinal tract image. The method is implemented by a computer or processor and comprises an image input step and a virtual intestinal tract cleansing step.
The image input step is a step in which a captured image, which is an image obtained by capturing a portion of a patient's large intestine, is input into a system for acquiring a virtually cleansed intestinal tract image. An example of the captured image is an image included in a three-dimensional captured image of the patient's large intestine.
The virtual intestinal tract cleansing step is a step in which the system obtains a virtually cleansed intestinal tract image. The virtually cleansed intestinal tract image is an image of a portion of the patient's large intestine from which residues included in the captured image have been removed. An example of the virtual intestinal tract cleansing step is a step of directly converting the captured image into the virtually cleansed intestinal tract image using a learning model. An example of the learning model is a model obtained by using, as teaching data, a residue-containing image that is an image of a portion of the large intestine including residues, and a residue-removed image that is an image of the portion of the large intestine after removing the residues, to obtain an image processing parameter for generating the residue-removed image from the residue-containing image.

This specification also describes a program for causing a computer to execute the method for acquiring a virtually cleansed intestinal tract image described above, as well as a computer-readable non-transitory information recording medium storing such a program.

This specification also provides a method for detecting a polyp from a captured image using a computer. The method includes a large intestine extraction step, which is a step of obtaining a large intestine image that is a partial image obtained by extracting only a large intestine part included in a captured image, which is an image obtained by capturing a portion of a patient's large intestine, and a polyp extraction step, which is a step of obtaining a polyp image that is an image obtained by extracting a polyp part included in the large intestine image. The method may further include a false positive removal step, which is a step of removing false positives included in the polyp image. The polyp extraction step may also serve as a cancer (colorectal cancer) extraction step. The false positive removal step may be a step of removing portions included in the polyp image that are incorrectly determined to be cancerous even though they are not cancer.

An example of the large intestine extraction step is a step of directly converting a captured image into a large intestine image using a learning model for extracting the large intestine. The learning model for extracting the large intestine is a model obtained by using, as teaching data, a large intestine ground-truth region image that is an image obtained by removing other organ parts than the large intestine from a certain captured image and extracting only the large intestine part. The large intestine ground-truth region image may be generated manually, for example, by a human inputting information into a computer, or may be generated automatically. The certain captured image is a captured image prepared in advance for constructing the learning model. Parameters for the learning model may be obtained from the teaching data to generate a trained learning model for extracting the large intestine.

An example of the polyp extraction step is a step of directly converting a large intestine image into a polyp image using a learning model. An example of the learning model used in the polyp extraction step is a model obtained by using, as teaching data, a certain large intestine image and a polyp ground-truth region image, which is an image obtained by extracting only a region of a polyp from the certain large intestine image. The certain captured image is a captured image prepared in advance for constructing the learning model. Parameters for the learning model may be obtained from the teaching data to generate a trained learning model for extracting polyps.

This specification also describes a program causing a computer to execute the method for acquiring a virtually cleansed intestinal tract image described above, as well as a non-transitory computer-readable storage medium storing such a program.

**Advantageous Effects of Invention**

The present invention can provide a method for acquiring a virtually cleansed intestinal tract image from a captured image of the large intestine obtained without using a laxative.

The present invention can provide a method for acquiring a virtually cleansed intestinal tract image capable of suppressing situations that interfere with the diagnosis of large intestine-related diseases.

According to the present invention, it is possible to perform a laxative-free colorectal CT examination using image processing based on virtual intestinal tract cleansing with machine learning.

The present invention can provide a method for detecting large intestine-related diseases, particularly polyps and colorectal cancer, from a captured image of the large intestine.

The present invention can provide a generative AI model for generating virtually cleansed intestinal tract images, as well as a generative AI model for detecting large intestine-related diseases.

**BRIEF DESCRIPTION OF DRAWINGS**

[FIG. 1] FIG. 1 is a flow diagram for explaining a method for acquiring a virtually cleansed intestinal tract image.

[FIG. 2] FIG. 2 is a block diagram illustrating a system for acquiring a virtually cleansed intestinal tract image.

[FIG. 3] FIG. 3 is a flowchart illustrating an example of the virtual intestinal tract cleansing step.

[FIG. 4] FIG. 4 shows an image obtained when virtual intestinal tract cleansing is actually performed using the system.

[FIG. 5] FIG. 5 is a flow diagram for explaining a method for detecting a large intestine polyp.

[FIG. 6] FIG. 6 is a block diagram illustrating a system for detecting a large intestine polyp.

[FIG. 7] FIG. 7 is a flowchart illustrating an example of the large intestine extraction step.

[FIG. 8] FIG. 8 is a flowchart illustrating an example of the polyp extraction step.

[FIG. 9] FIG. 9 is a flowchart illustrating an example of the false positive removal step.

[FIG. 10] FIG. 10 shows an image obtained when large intestine extraction is actually performed using the system.

[FIG. 11] FIG. 11 shows examples of virtual endoscopic image displays and cross-sectional image displays of polyp ground-truth region images used in the training and test phases of the learning model for polyp extraction.

[FIG. 12] FIG. 12 shows examples of virtual endoscopic image displays and cross-sectional image displays of polyp images output from different trained learning models for polyp extraction.

[FIG. 13] FIG. 13 shows an image obtained when false positive removal is actually performed using the system.

## DESCRIPTION OF EMBODIMENTS

Embodiments for carrying out the present invention will be described below with reference to the drawings. The present invention is not limited to the embodiments described below, and includes modifications made by those skilled in the art within a range that is obvious from the following embodiments.

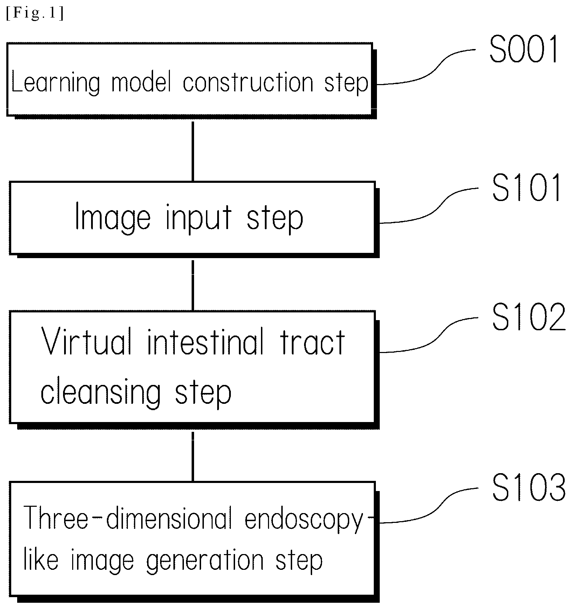

FIG. 1 is a flow diagram for explaining a method for acquiring a virtually cleansed intestinal tract image. As shown in FIG. 1, the method includes an image input step (S101) and a virtual intestinal tract cleansing step (S102). Here, "S" denotes a step. The method is implemented by a computer or a processor. The method can be considered as a laxative-free colorectal CT examination using image processing based on virtual intestinal tract cleansing with machine learning.

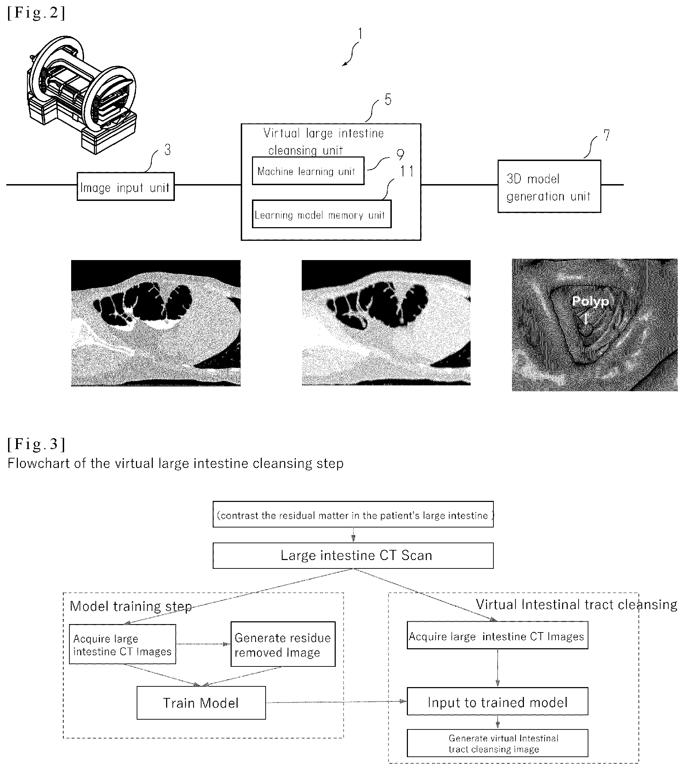

FIG. 2 is a block diagram illustrating a system for acquiring a virtually cleansed intestinal tract image. As shown in FIG. 2, the system 1 for acquiring a virtually cleansed intestinal tract image includes an image input unit 3 and a virtual intestinal tract cleansing unit 5. The system is implemented by a computer or a processor. The system 1 includes a processor and a memory storing a program. The program may be configured to cause the processor to execute each step of the method for acquiring a virtually cleansed intestinal tract image. As shown in FIG. 2, the system may further include one or two or more of a three-dimensional model generation unit 7, a machine learning unit 9, and a learning model memory unit 11.

The computer includes an input unit, an output unit, a control unit, a computation unit, and a memory unit, each of which is connected by a bus or the like to enable information exchange. For example, the memory unit may store a control program and/or various types of information. When predetermined information is input from the input unit, the control unit reads the control program stored in the memory unit. The control unit appropriately reads information stored in the memory unit and transmits it to the computation unit. The control unit also transmits, as needed, the information input from the input unit to the computation unit. The computation unit performs computation steps using the received information and stores the results in the memory unit. The control unit reads the computation results stored in the memory unit and outputs them via the output unit. In this manner, various processes and steps are executed. These steps may be performed by the respective units or means. The computer may include a processor that implements various functions and steps. The computer may operate in a standalone configuration, or one or more functions may be distributed between a server and a terminal. In such a case, the server and terminal are preferably capable of exchanging information via a network such as the Internet or an intranet. The computer may include a processor and a memory connected to the processor. The memory may store instructions, and when the instructions are executed by the processor, the computer performs various steps and functions as the respective units. The computer may construct a learning model by providing various teaching data and perform computation using machine learning. In this case, the computer may perform various analyses and steps using a learning model created by AI (artificial intelligence) machine learning or deep learning.

The image input step (S101) is a step in which a captured image, which is an image obtained by capturing a portion of a patient's large intestine, is input to the system for acquiring a virtually cleansed intestinal tract image. An example of the captured image is an image included in a three-dimensional captured image of the patient's large intestine. The captured image may also be a medical image obtained by imaging the patient's abdomen. By using this system, an image of a portion of the patient's large intestine from which residue has been removed can be obtained. Therefore, the patient does not need to ingest a laxative to remove residue (feces) from the large intestine. Accordingly, an example of the captured image is a medical image of the patient in a normal state (without ingestion or administration of a laxative). In this manner, the captured images used in the present invention may be medical images in which intestinal residue has not been physically removed. This can significantly reduce the burden on the patient. A three-dimensional captured image of the large intestine is formed by a plurality of cross-sectional images of the large intestine. An example of such a three-dimensional captured image is a CT scan image. Examples of the captured image include one or more of such cross-sectional images of the large intestine.

For example, the system 1 is connected to a CT scanning system. When a CT scan of a portion of the patient's large intestine is performed, the CT scanning system stores the CT scan image of the patient's large intestine portion. The CT scan image stored in the CT scanning system is output from the output unit of the CT scanning system to the system 1. The image input unit 3 of the system 1 receives the CT scan image. In this manner, a captured image (an image obtained by capturing a portion of the patient's large intestine) is input to the system 1. As CT scanning continues, multiple captured images may be input to the system 1. The captured images input to the system 1 may be stored in the memory of the system 1 as appropriate. In addition to the captured images, information regarding the patient may also be input to the system 1. Examples of information regarding the patient include one or more of the patient identification number (ID), the patient's name, the patient's sex, the patient's age, the patient's medical history, information regarding the patient's alcohol consumption, and information regarding colorectal cancer in the patient's relatives.

Figure 3 is a flowchart illustrating an example of the virtual intestinal tract cleansing step. The virtual intestinal tract cleansing step (S102) is a step in which the system 1 obtains a virtually cleansed intestinal tract image. The virtually cleansed intestinal tract image is an image of a portion of the patient's large intestine obtained by removing the part that corresponds to residue in the large intestine included in the captured image. An example of the virtual intestinal tract cleansing step is a step of directly converting the captured image into the virtually cleansed intestinal tract image using a learning model. An example of the learning model is a trained model obtained by using, as teaching data, a residue-containing image that is an image of a portion of the large intestine including residue and a residue-removed image that is an image of the portion of the large intestine after removing the residue, and by obtaining image processing parameters for generating the residue-removed image from the residue-containing image. This model can be continuously updated by adding additional teaching data. As shown in the examples, there is a correlation between the residue-containing image and the residue-removed image, and therefore, a learning model can be constructed by using them as teaching data. For example, the virtual intestinal tract cleansing unit 5 reads the captured image from the memory and, based on instructions from the program, the arithmetic unit performs image processing using the learning model to remove the residue included in the captured image and obtain the virtually cleansed intestinal tract image. The virtual intestinal tract cleansing step (S102) preferably directly converts a CT scan image volume of the large intestine into a corresponding virtually cleansed intestinal tract image volume using the learning model. The obtained virtually cleansed intestinal tract image may be stored in the memory as appropriate. As shown in the examples described later, the learning model allows obtaining the virtually cleansed intestinal tract image. By improving machine learning, it is possible to obtain the captured images and virtually cleansed intestinal tract images of a patient without administering not only a laxative but also a contrast agent. In this case, a trained model may be constructed using, as teaching data, captured images of a patient without administering a laxative or a contrast agent and captured images obtained when a laxative and a contrast agent are administered, and the constructed trained model may be used to obtain a virtually cleansed intestinal tract image from the captured images of the patient without administering a laxative or a contrast agent.

Three-dimensional endoscopy-like image generation step (S103) The three-dimensional endoscopy-like image generation step is a step of obtaining a three-dimensional model image representing the patient's large intestine using multiple virtually cleansed intestinal tract images. Image processing is performed for each captured image by the virtual intestinal tract cleansing step (S102), and a virtually cleansed intestinal tract image, in which the residue has been virtually removed, is obtained. The three-dimensional endoscopy-like image generation step may be performed by the three-dimensional model creation unit 7 of the system 1. The three-dimensional model creation unit 7 can generate a model of the patient's large intestine using virtually cleansed intestinal tract images of the necessary portions of the large intestine. Creating a three-dimensional model of the large intestine using multiple CT scan images of the large intestine is publicly known. Therefore, the three-dimensional model creation unit 7 may be implemented using a publicly known program. The model of the patient's large intestine obtained in this manner is equivalent to a model obtained from an endoscopic examination after physically removing residue by administering a laxative. By using this model, images of the patient's large intestine from various angles can be obtained, and the large intestine can be visually observed in three dimensions. Therefore, an image of the patient's large intestine generated based on the three-dimensional model is referred to as a three-dimensional endoscopy-like image. The memory of the system 1 may store the obtained model of the patient's large intestine as appropriate. The system 1 may read the model of the large intestine from the memory and output required cross-sectional images to an output unit such as a monitor.

Learning model construction step (S001)

The learning model construction step is a step of constructing and developing (training) a machine learning model used for virtual intestinal tract cleansing. This step may be performed, for example, by the machine learning unit 9 of the virtual intestinal tract cleansing unit 5. The machine learning unit 9 may train the learning model using as teaching data, for example, CT scan images of multiple patients after intestinal cleansing (e.g., CT scan images after administration of a laxative) and normal CT scan images of the same patients. By using such a learning model, the model can be applied to a patient's normal CT scan image to obtain a virtually cleansed image in which residue included in the image has been removed, i.e., a virtual intestinal tract cleansing image, through image processing. The constructed and trained learning model (trained model) may be stored in the learning model memory unit 11 and updated as appropriate.

A learning model may be trained by administering an oral contrast agent to a patient, imaging the residue in the large intestine, acquiring CT images of the large intestine, and generating residue-removed images using the learning model. The construction and training of the learning model may also be performed using a diffusion model, as described later.

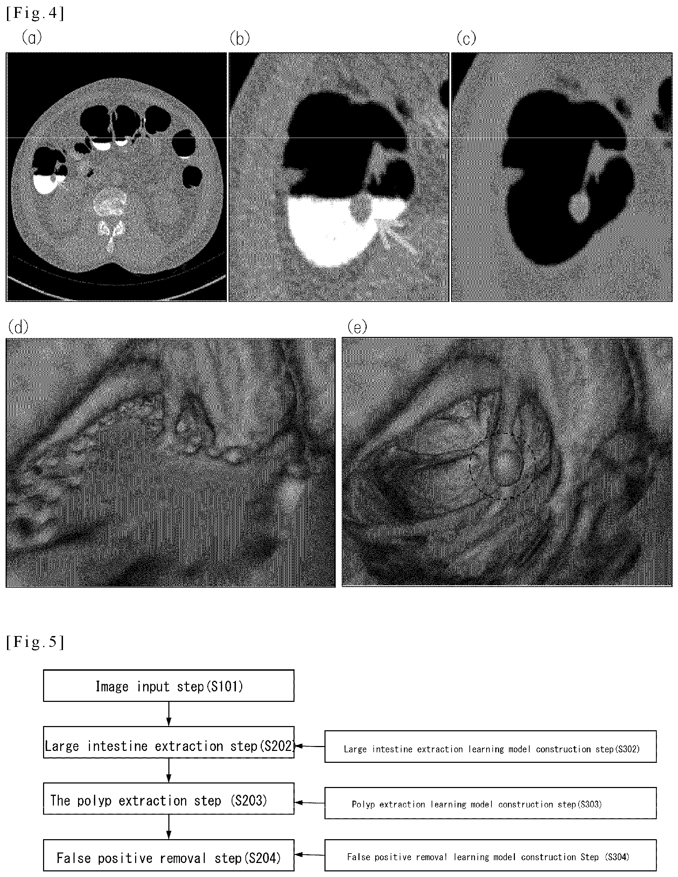

FIG. 5 is a flowchart illustrating an example of a method for detecting colorectal polyps. As shown in FIG. 5, this method example includes an image input step (S101), a large intestine extraction step (S202), a polyp extraction step (S203), and a false positive removal step (S204), where "S" indicates a step. This method is implemented by a computer or processor. The method may be regarded as a computer-assisted diagnosis for a nonlaxative colorectal CT examination using a method for detecting colorectal polyps based on image processing with a learning model. In this method, the computer executes a large intestine extraction step for obtaining a large intestine image, which is a partial image extracted from a captured image representing a portion of the patient's large intestine. The computer also executes a polyp extraction step for obtaining a polyp image, which is an image extracted from the large intestine image that includes only the polyp region. This constitutes a method for detecting colorectal polyps. The method may further include a false positive removal step, which removes false positives included in the polyp image. The processor may include a memory storing instructions, and the processor causes the computer to perform the above steps according to the instructions stored in the memory.

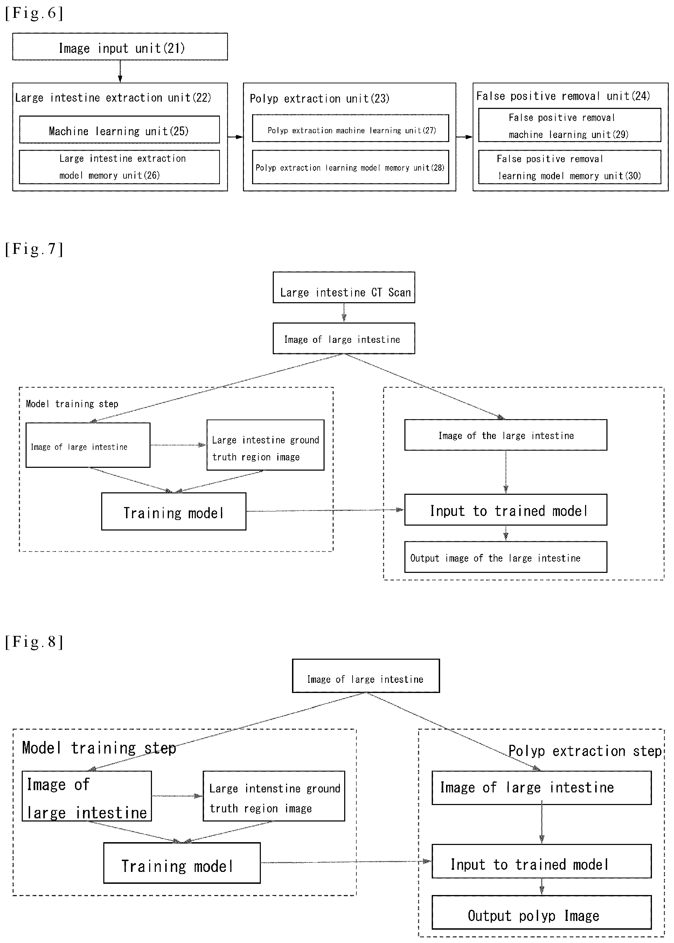

FIG. 6 is a block diagram illustrating an example of a system for detecting colorectal polyps. As shown in FIG. 6, the system for detecting colorectal polyps includes an image input unit 21, a large intestine extraction unit 22, a polyp extraction unit 23, and a false positive removal unit 24. The large intestine extraction unit 22 includes a large intestine extraction machine learning unit 25 and a large intestine extraction model memory unit 26. The polyp extraction unit 23 includes a polyp extraction machine learning unit 27 and a polyp extraction model memory unit 28. The false positive removal unit 24 includes a false positive removal machine learning unit 29 and a false positive removal model memory unit 30. The system is implemented by a computer or processor. The system includes a processor and a memory storing a program. The program may be configured to cause the processor to execute each step of the method for detecting colorectal polyps.

Image Input Step (S101)

The image input step is the same as described above.

Figure 7 is a flowchart illustrating an example of the large intestine extraction step. The large intestine extraction step (S202) is a step in which the system obtains a large intestine image. The large intestine image is an image in which only the large intestine portion included in the captured image is extracted. The large intestine image may also be an image in which only the large intestine portion included in a virtual bowel cleansing image is extracted from the virtual bowel cleansing image. An example of the large intestine extraction step is a step in which a learning model is used to directly convert the captured image (or virtual bowel cleansing image) into a large intestine image. An example of the learning model is a model obtained using as training data a pair of images consisting of a captured image (or virtual bowel cleansing image) including the patient's large intestine and an image obtained by removing portions other than the large intestine from the captured image (or virtual bowel cleansing image). This model may also be a model obtained by acquiring image processing parameters for obtaining a large intestine image from the captured image (or virtual bowel cleansing image). The captured image used when constructing this learning model is a captured image prepared for the purpose of constructing the learning model. The captured image may also be a virtual bowel cleansing image. For example, the large intestine extraction unit 22 reads a captured image or virtual bowel cleansing image from the memory unit and, based on instructions from the program, the large intestine extraction machine learning unit 25 performs image processing using the large intestine extraction learning model to extract only the large intestine region included in the captured image or virtual bowel cleansing image, thereby obtaining the large intestine image. The large intestine extraction step (S202) preferably directly converts a large intestine CT scan image volume into a corresponding large intestine image volume using the learning model. The obtained large intestine image may be stored in the memory unit as appropriate.

The large intestine Extraction Learning Model Construction Step (S302)

The large intestine extraction learning model construction step is a step in which a machine learning model used in the large intestine extraction step is constructed and developed (trained). This step may be performed, for example, by the machine learning unit 25 of the large intestine extraction unit 22. The machine learning unit 25 may train a learning model using, as training data, CT scan images of multiple patients and corresponding large intestine ground truth region images in which portions other than the large intestine have been removed, leaving only the large intestine region. By using such a learning model optimized for large intestine extraction, the learning model can be applied to a CT scan image including a patient's large intestine to obtain, through image processing, a large intestine image in which only the large intestine included in the captured image is extracted. The constructed and trained learning model (trained model) may be stored in the learning model memory unit 26 and updated as appropriate.
The construction of the large intestine extraction learning model may also be performed using a diffusion model. If the large intestine region includes polyps, the large intestine image may also include those polyps.

Furthermore, the training data of captured images used for training the large intestine extraction learning model may include additional abdominal images generated using the diffusion model described in the following examples.

Figure 8 is a flowchart illustrating an example of the polyp extraction step. The polyp extraction step (S203) is a step in which the system obtains a polyp image. The polyp image is an image obtained by extracting only the polyp regions included in the large intestine image from the large intestine image. An example of the polyp extraction step is a step in which a learning model is used to directly convert a large intestine image into a polyp image. An example of the learning model is a model obtained by using a large intestine image, which is an image of the patient's extracted large intestine, and a polyp ground-truth region image, which is an image obtained by extracting only the polyp regions included in that large intestine image, as training data. The polyp ground-truth region image may be created manually or generated automatically. This model may also be a model obtained by acquiring image processing parameters for obtaining a polyp image from a large intestine image. For example, the polyp extraction unit 23 reads a large intestine image or a virtual bowel-cleansed image from the memory, and based on program instructions, the polyp extraction machine learning unit 27 uses the polyp extraction learning model to perform image processing that extracts only the polyp regions included in the large intestine image or virtual bowel-cleansed image, thereby obtaining a polyp image. It is preferable that the polyp extraction step (S203) directly converts a large intestine image (or large intestine image volume) into a corresponding polyp image (or polyp image volume) using the polyp extraction learning model. The obtained polyp image may be stored in the memory as appropriate. The polyp extraction step may also be a cancer (colorectal cancer) extraction step.

Polyp Extraction Learning Model Construction Step (S303)

The polyp extraction learning model construction step is a step for constructing and developing (training) a machine learning model used in the polyp extraction step. This step is, for example, performed by the polyp extraction machine learning unit 27 of the polyp extraction unit 23. The machine learning unit 27 may train the polyp extraction learning model using as training data a plurality of patients' large intestine images and polyp ground-truth region images, which are images obtained by extracting only the polyp regions contained in those large intestine images. The polyp ground-truth region images may be created manually or generated automatically. By using such a learning model optimized for polyp extraction, a polyp image can be obtained by applying the learning model to a large intestine image and extracting the polyps contained therein through image processing. The constructed and trained learning model (trained model) may, for example, be stored in the polyp extraction learning model memory unit 28 and updated as needed. The construction of the polyp extraction learning model may be performed using a diffusion model as described in the following examples. Additionally, the large intestine image training data used for training the polyp extraction learning model may include large intestine images generated using the diffusion model described in the following examples.

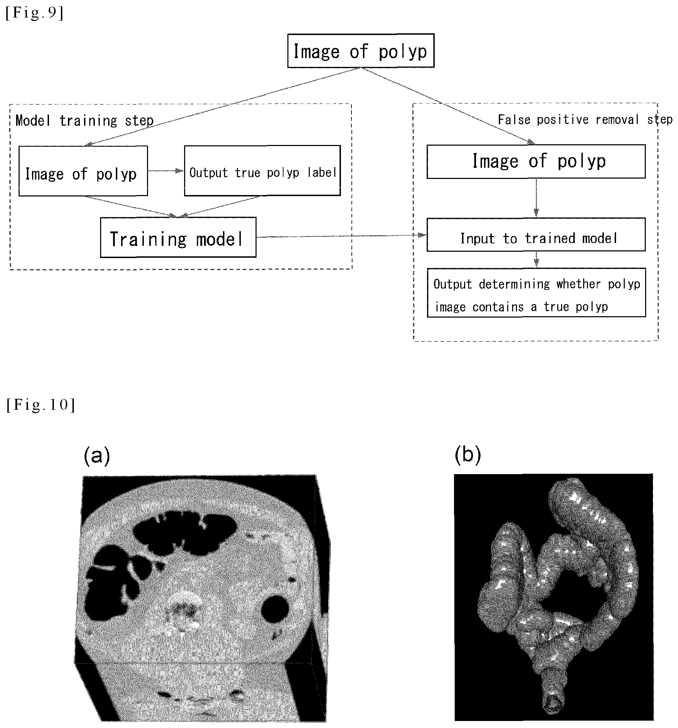

Figure 9 is a flowchart illustrating an example of the false positive removal step. The false positive removal step (S204) is a step in which the system removes false positives from a polyp image to obtain an image containing only true polyps. A false positive may be a portion of an image included in the polyp image even though it is not a polyp, or a portion of a polyp image incorrectly identified as cancer even though it is not cancer. An example of the false positive removal step is a step in which a false positive removal learning model is used to remove portions corresponding to false positives from a polyp image. The learning model may be obtained by using as training data a set of polyp images and labels indicating whether each image contains a true polyp, thereby obtaining image processing parameters for determining whether a polyp image contains a true polyp. For example, the false positive removal unit 24 reads the polyp image from the memory, and based on program instructions, the false positive removal machine learning unit 29 applies the false positive removal learning model to perform image processing to determine whether the polyp image contains a true polyp. If the polyp is not a true polyp, the system removes that polyp (i.e., the portion of the polyp image that is a false positive). The false positive removal learning model is stored in the false positive removal learning model memory unit 30 and may be read and used as needed. The portions of polyps that are not removed (true polyp portions) may be stored in the memory as appropriate. The false positive removal step may also be a step for removing portions in a polyp image that are incorrectly identified as cancer even though they are not cancer.

False Positive Removal Learning Model Construction Step (S304)

The false positive removal learning model construction step is a step for constructing and developing (training) a machine learning model used in the false positive removal step. This step may, for example, be performed by the false positive removal machine learning unit 29 of the false positive removal unit 24. The false positive removal machine learning unit 29 may train the learning model using, as training data, polyp images from multiple patients along with identification information (labels) indicating whether each polyp image corresponds to a true polyp. By using such a learning model optimized for false positive removal, the learning model can be applied to polyp images to determine whether the polyp image contains a true polyp. The constructed and trained learning model (trained model) may be stored in the learning model memory unit 30 and may be updated as needed. The construction of the false positive removal learning model may also be performed using the diffusion model described in the embodiments below. Furthermore, the polyp image training data used to train the false positive removal learning model may include polyp images generated using the diffusion model described in the embodiments below.

The false-positive removal model may use the size of a polyp region in a polyp image as a threshold. For example, the size of the polyp region (the maximum three-dimensional diameter of the polyp) may be used as the threshold. Examples of the threshold may be any value of 6 mm or more and 10 mm or less, or any value of 6.5 mm or more and 9.5 mm or less.

This specification also describes a program that causes a computer to execute the above-described method for acquiring a virtually cleansed intestinal tract image, as well as a non-transitory computer-readable storage medium storing such a program. Examples of the non-transitory information recording medium include a CD-ROM, a DVD, and a USB memory.

**Example Embodiments**

[Example 1]

Development of Learning Model (DDP Model)

In the following, a basic denoising diffusion probabilistic model (DDP model) for generating synthetic high-quality image volumes was constructed. Training of the DDP model includes minimizing the divergence between a target distribution and a distribution of generated images, and maximizing the likelihood of the observed training data. The diffusion model is a latent variable model represented by the following equation.

[Formula 1]

$$p_\theta(x_0) = \int p_\theta(x_{0:T}) \mathrm{d}x_{1:T}$$

$x_1, ..., x_T$ are latent variables having the same dimensionality as the data $x_0 \sim q(x_0)$.
The joint distribution is given by the following equation:

[Formula 2]

$$p_\theta(x_{0:T})$$

is referred to as the reverse step and is defined as a Markov chain with learned Gaussian transitions. The learned Gaussian transitions are given by the following equation:

[Formula 3]

$$p_\theta(x_{0:T}) = p(x_T) \prod_{t=1}^{T} p_\theta(x_{t-1}|x_t)$$

$$p_\theta(x_{t-1}|x_t) = N(x_{T-1}; \mu_\theta(x_t, t), \Sigma_\theta(x_t, t)).$$

The starting point is as follows:

[Formula 4]

$$p(x_T) = N(x_T; 0, I)$$

The diffusion model differs from other latent variable models in terms of the approximate posterior distribution, that is, the forward step or diffusion step. The approximate posterior distribution is expressed by the following equation:

[Formula 5]

$$q(x_{1:T}|x_0)$$

This approximate posterior distribution is fixed as a Markov chain that progressively adds Gaussian noise to the data. Using the forward step, $x_t$ at any time step $t$ can be sampled in closed form. Next, we denote the following:

[Formula 6]

$$\alpha_t = 1 - \beta_t \qquad \text{and} \qquad \bar{\alpha}_t = \prod_{s=1}^{t} \alpha_s$$

Then, the approximate posterior distribution can be expressed as:

[Formula 7]

$$q(x_t|x_0) = N(x_t; \sqrt{\bar{\alpha}_t} x_0, (1 - \alpha_t)I)$$

Training is performed by optimizing the standard variational lower bound with respect to the negative log-likelihood. To implement the DDP model, the variance of the forward step was set as a constant. These variances were increased linearly from $\beta_1 = 10^{-4}$ to $\beta_T = 0.02$, where $\alpha t = 1 - \beta_t$. To represent the reverse step, an nD U-Net with group normalization throughout was used as the backbone. Parameters were shared across different time steps, and a transformer sinusoidal positional embedding was used to specify the time step to the network.

Development of the Learning Model (Diffusion-based nD U-Net Model)

The nD U-Net diffusion model can be trained for image (direct) translation tasks by pairing input image volumes (e.g., MRI or CT scans) with their corresponding ground truth images. By introducing noise into the input image volumes during training, the model learns to explore various perturbations and construct a diffusion embedding representation that captures the underlying image features. This representation is then transformed into the corresponding ground truth image.

In this embodiment, a Diff-UNet model, described below, was used.

In the case of three-dimensional (3D) data, let $V$ denote the input image volume, which can be represented as follows:

[Formula 8]

$$V \in R^{M \cdot D \cdot W \cdot H}$$

Here, M denotes the number of relevant image modalities. First, the corresponding single-channel, multi-label input map of size $D \times W \times H$ is converted into a multi-channel label map $x_0$ via one-hot encoding. The label map $x_0$ can be represented as follows:

[Formula 9]

$$x_0 \in R^{N \cdot D \cdot W \cdot H}$$

Here, $N$ denotes the number of labels. Next, to obtain the noisy label map $x_t$, forward diffusion was applied, adding noise $\varepsilon$ over $t$ consecutive steps.

To recover the original input map, $V$ and $x_t$ were concatenated along the channel dimension and fed into the encoder, allowing the extraction of a multi-scale feature volume by the denoising $nD$ UNet. The multi-scale feature volume is represented by the following equation:

[Formula 10]

$$\hat{I}_f$$

To introduce raw volumetric image features, multi-scale feature volumes were extracted from intensity data through a feature encoder of the same size. The multi-scale feature volume is represented by the following equation:

[Formula 11]

$$\tilde{i}_f$$

By summing the multi-scale feature volume with the multi-scale feature volume extracted from intensity data, a fused multi-scale feature is obtained and supplied to the decoder. This enables the prediction to be obtained. The prediction is represented by the following equation:

[Formula 12]

$$\ddot{x}_0 = R^{N \cdot D \cdot W \cdot H}$$

The Diff-UNet model can be trained using a loss function. The loss function is represented by the following equation:

[Formula 13]

$$L_{total} = L_{Dice}(\ddot{x}_0,\ x_0) + L_{BCE}(\ddot{x}_0,\ x_0) + L_{MSE}(\ddot{x}_0,\ x_0)$$

Here, $L_{Dice}$ is the Dice loss, $L_{BCE}$ is the binary cross-entropy loss, and $L_{MSE}$ is the mean squared error loss.

In the testing phase, the diffusion steps were repeated $t$ times using the denoising diffusion implicit (DDI) model approach. To improve the robustness of segmentation, the final output is determined based on the number of prediction steps and the associated uncertainty. This uncertainty is estimated similarly to the Monte Carlo (MC) dropout method. In this approach, MC dropout activates the dropout layers and performs $S$ forward passes to estimate an uncertainty map. By initializing the random noise $x_t$ during the testing phase, Diff-UNet introduces randomness without needing to activate dropout. Diffusion is performed over $t$ steps, with $S$ outputs predicted at each step, which are used to calculate uncertainty. The uncertainty is computed using the following equation.

[Formula 14]

$$u_i = -p_i \log(p_i)$$
$$p_i = 1/S \ \sum p_{is}$$
$$s = 1 \ldots S$$

The fusion weight for combining the number of prediction steps and uncertainty is calculated using the following equation.

[Formula 15]

$$w_i = \exp(\sigma(i/\text{scale}) \times (1 - u_i))$$

Here, $\sigma$ is the sigmoid function, i is the current prediction step, and u is the uncertainty matrix. By applying the weight w to the prediction results at each step, the final fused result can be obtained. The final fused output is represented by the following equation.

[Formula 16]

$$Y = \sum w_i \times p_i$$
$$i = 1 .. t,$$

Virtual Bowel Cleansing

Figure 4 shows images obtained when performing virtual bowel cleansing using the system.

Figure 4(a) is a photograph corresponding to the clinical CT scan image obtained using a contrast agent. In Figure 4(a), the polyp is submerged in liquid filled with the contrast agent. Figure 4(b) is an enlarged view of the polyp region. Figure 4(c) shows an image obtained when virtual bowel cleansing was actually performed using the system. Figure 4(d) is a cross-sectional image of the large intestine created as a 3D model based on the CT scan image in which the polyp is submerged in liquid filled with the contrast agent. In this example, due to residual liquid obstructing the view, the polyp was not visible in the virtual endoscopic image. Figure 4(e) is a cross-sectional image of the large intestine created as a 3D model based on the virtual bowel cleansing image. In this example, the polyp can be seen in the area indicated by the dotted line.

Quantitative Evaluation of Virtual Bowel Cleansing

To quantitatively evaluate the image quality of the virtual bowel cleansing images, a custom anthropomorphic large intestine phantom (Anthropomorphic the large intestine Phantom, Phantom Laboratory, Salem, NY, USA) was used. A solution was prepared as simulated contrast residue by mixing 300 mL of saline and 10 g of cereal with 40 mg/mL of non-ionic iodine contrast agent (Omnipaque, GE Healthcare, Chicago, IL, USA) at 10 mg. This simulated contrast residue was injected into the phantom. Using a multi-row CT scanner (SOMATOM Definition Flash, Siemens Healthcare, Erlangen, Germany) with a tube voltage of 120 kVp, tube current of 236 mA, and slice thickness of 0.6 mm, a CT scan image volume of the large intestine phantom was obtained. From this CT scan volume, 100 volumes of interest (VOIs) measuring $128 \times 128 \times 128$ voxels, each containing a portion of the large intestine, were extracted. These VOIs were then directly converted into virtual bowel cleansing image volumes using the three types of trained models described below.

The first trained model is a three-dimensional (3D) extension of the conventional U-Net, known as 3D U-Net. This 3D U-Net is a convolutional neural network that consists of two symmetric paths: an encoder path composed of convolutional network layers to capture the local context of the input image volume, and a decoder path composed of transposed convolution layers to enable precise localization. In the 3D U-Net, skip connections are used to connect the corresponding encoder and decoder layers to preserve spatial information that may be lost during the downsampling steps of the encoder path.

The second trained model is the UNet Transformer (UNETR). Recently, transformers utilizing self-attention have attracted attention as an alternative to conventional convolutional neural networks. UNETR incorporates a transformer into the encoder path of the 3D U-Net described above in order to capture long-range dependencies within the input image volume. This allows the model to learn relationships between all local patches in the input image volume, resulting in superior image processing performance compared to traditional convolutional networks.

The third trained model is a generative AI model, a type of diffusion-based nD U-Net, specifically the Diff-UNet described above. Diff-UNet introduces noise into the input image volume and then generates the virtual bowel cleansing image volume through an iterative denoising step using a denoising diffusion probabilistic model.

The virtual bowel cleansing image volumes obtained by applying these three trained models to the 100 VOIs described above were compared with the CT scan image volume of the large intestine phantom before the injection of contrast residue. The difference was measured using the peak signal-to-noise ratio (PSNR). As a result, the mean $\pm$ standard deviation of the PSNR values for 3D U-Net, UNETR, and Diff-UNet were $28.70 \pm 3.00$, $29.88 \pm 2.53$, and $30.60 \pm 1.81$, respectively.

These results indicate that, regardless of which trained model is used, the CT scan image volume can be converted into a high-quality virtual bowel cleansing image volume. Furthermore, when using the generative AI model Diff-UNet, it is possible to generate virtual bowel cleansing image volumes with superior image quality compared to those obtained using non-generative AI models.

[Example 2]

The Large Intestine Extraction

FIG. 10 shows images obtained when performing large intestine extraction using the system for detecting colorectal polyps.

FIG. 10(a) is a CT scan image volume obtained from clinically acquired CT images of a patient's large intestine. In FIG. 10(a), the entire abdominal image volume, including the large intestine, is displayed. FIG. 10(b) shows the large intestine image obtained when performing large intestine extraction using the system, presented in a three-dimensional view.

[Example 3]

Polyp Extraction

In this example, the performance of three types of polyp extraction learning models was compared in the polyp extraction step, where large intestine images are converted into polyp images. Here, the polyp extraction learning models used were the previously described 3D U-Net, UNETR, and the generative AI model Diff-UNet.

In this example, CT scan images of the large intestine from 150 cases obtained from multiple clinical trials of large intestine cancer screening via large intestine CT examinations were used to create training data for the polyp extraction learning models. In these cases, residual matter was enhanced using orally administered iodine or barium contrast agents, and each case was scanned in two of the supines, prone, or lateral positions. Across all cases, 11 different CT scanners were used, with tube voltages of 120 kVp or 140 kVp, pixel spacing of 0.54-0.97 mm, and slice thickness of 0.75-5.00 mm.

The training data were manually created by experienced radiologists, who extracted only the polyp regions from the large intestine CT images using corresponding colonoscopy and CT examination reports for each case. To do this, the CT scan images were first interpolated to image volumes with isotropic spatial resolution of 0.625 mm. Then, for each known polyp within the image volumes, a $48\times48\times48$ voxel volume of interest (VOI) centered on the polyp was extracted, and the polyp region was manually segmented using 3D Slicer (slicer.org). As a result, a total of 946 VOIs, each containing a single polyp, along with their corresponding polyp ground-truth images, were obtained as the training dataset.

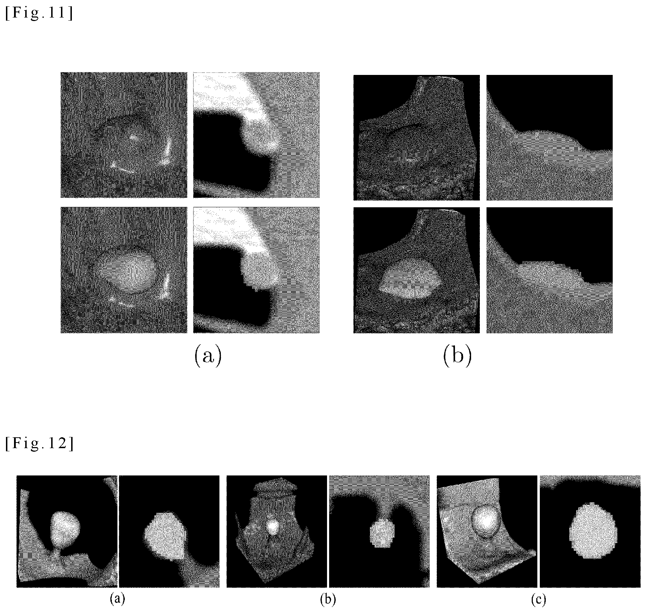

Figure 11(a) shows examples of VOIs included in the training data in the upper row, and in the lower row, the corresponding polyp ground-truth images overlaid in white.

Each polyp extraction learning model was constructed using MONAI (monai.io) and PyTorch (pytorch.org). The training of the polyp extraction learning models was performed on a 32 GB Tesla V100S GPU (NVIDIA, Santa Clara, CA). During training, the above-described training data were first split into 80% training data and 20% validation data, and data augmentation was applied to the training data through random Gaussian noise addition, random flipping, 90-degree rotation, and scaling. Next, the weights of the polyp extraction learning models were initialized with random values, and model training using the training data and performance evaluation of polyp extraction using the validation data were repeated 1,000 times. The performance of polyp extraction was evaluated using the Dice score between the polyp ground-truth image corresponding to each VOI in the validation data and the polyp image output by the polyp extraction learning model when the VOI was input. For each polyp extraction learning model, the model that achieved the highest Dice score among the 1,000 iterations was selected as the trained model to be used in the test phase described below.

As test data for the test phase, a custom anthropomorphic large intestine phantom containing simulated polyps (Anthropomorphic Large intestine Phantom, Phantom Laboratory, Salem, NY, USA) was scanned using a 16-row photon-counting CT scanner (OmniTom, NeuroLogica, MA, USA) with the following parameters: tube voltage 120 kVp, tube current 40 mA, slice thickness 0.71 mm, and X-ray photon-counting threshold 50 keV. The images were reconstructed using a filtered back-projection method with a spatial resolution of 0.6 mm and a reconstruction interval of 0.6 mm. The resulting CT scan images were interpolated to a CT scan image volume with isotropic spatial resolution of 0.625 mm, and 17 VOIs ($48 \times 48 \times 48$ voxels) centered on the simulated polyps were extracted. The regions of the simulated polyps contained in these VOIs were manually delineated using 3D Slicer to create polyp images, which were used as the polyp ground-truth images corresponding to the VOIs.

Figure 11(b) shows examples of VOIs contained in the test data in the top row, and in the bottom row, the corresponding polyp ground-truth images overlaid in white.

The polyp extraction performance of each trained polyp extraction model in the test phase was evaluated using the Dice score between the polyp images generated by the trained models from each VOI in the test data and the corresponding polyp ground-truth images for those VOIs. As a result, the mean $\pm$ standard deviation of the Dice scores were as follows: $0.738 \pm 0.14$ for 3D U-Net, $0.745 \pm 0.14$ for UNETR, and $0.751 \pm 0.15$ for Diff-UNet. Among these three polyp extraction models, Diff-UNet achieved the highest mean Dice score, followed by UNETR. Furthermore, for all polyps contained in the test data, Diff-UNet achieved the highest Dice score of 0.946. Figure 12 shows examples of virtual endoscopic images and cross-sectional images of polyps obtained from each trained polyp extraction model. In all models, pedunculated polyps, as shown in the figure, exhibited the highest Dice scores.

Figure 12(a) shows an example of a polyp image obtained from 3D U-Net. The Dice score in this example was 0.88.

Figure 12(b) shows a polyp image output from UNETR. The Dice score in this example was 0.88.

Figure 12(c) shows a polyp image output from Diff-UNet. The Dice score in this example was 0.88.

These results demonstrate that all polyp extraction models can accurately extract polyp regions from large intestine CT scan images to generate polyp images. In particular, using the generative AI model Diff-UNet enables the generation of polyp images with higher performance compared to polyp extraction models that do not use generative AI.

[Example 4]

Development of the Learning Model (Improved Diffusion-based nD U-Net Model)

The diffusion-based nD U-Net model described above was improved as follows to construct a false-positive removal learning model.

First, the diffusion-based nD U-Net model was constructed and trained using the method described in Example 3. From the resulting trained model, the encoder portion of the U-Net was extracted, and a binary classification convolutional network was connected to the bottleneck layer to form a deep network. This deep network was trained using the VOIs included in the training data described in Example 3 together with the labels indicating whether the polyps contained in the VOIs are true polyps. By training in this manner, a false-positive removal learning model was constructed.

False-Positive Removal

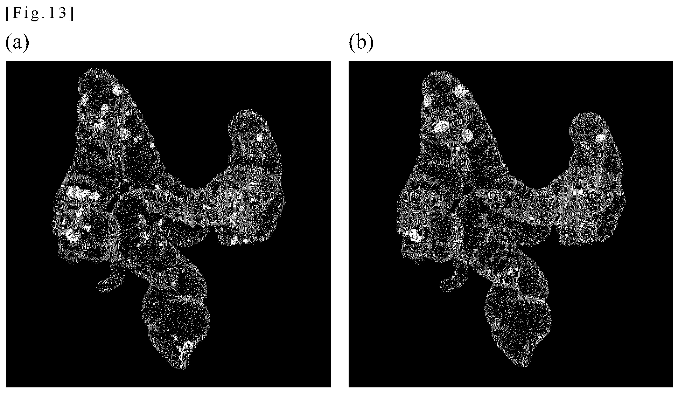

Figure 13 shows images obtained when false-positive removal was performed using the above-described learning model.

Figure 13(a) shows the polyp regions extracted from the large intestine image, such as that shown in Figure 10(b), overlaid in white on a semi-transparent large intestine image. Figure 13(b) shows the polyp regions that remained after false-positive removal. Most of the polyps shown in Figure 13(a) were eliminated, leaving only the true polyps detected.

**INDUSTRIAL APPLICABILITY**

This invention can be utilized in the field of medical devices.

**REFERENCE SIGNS LIST**

1. System
3. Image Input Unit
5. Virtual Large intestine Cleansing Unit
7. 3D Model Generation Unit
9. Machine Learning Unit
11. Learning Model Memory Unit
21. Image Input Unit
22. Large intestine Extraction Unit
23. Polyp Extraction Unit
24. False Positive Removal Unit
25. Machine Learning Unit for Large intestine Extraction
26. Learning Model Memory Unit for Large intestine Extraction
27. Polyp Extraction Machine Learning Unit
28. Polyp Extraction Learning Model Memory Unit
29. False Positive Removal Machine Learning Unit
30. False Positive Removal Learning Model Memory Unit

**Claims**

1.    A method for acquiring a virtually cleansed intestinal tract image, the method comprising:

inputting a captured image that is an image obtained by capturing a portion of a large intestine of a patient; and
obtaining a virtually cleansed intestinal tract image that is an image of the portion of the large intestine of the patient after removing a part that corresponds to residue in the large intestine included in the captured image.

2.    The method according to claim 1, wherein
the captured image is an image included in a three-dimensional captured image of the large intestine of the patient.

3.    The method according to claim 2, wherein
obtaining a virtually cleansed intestinal tract image uses a learning model to directly convert the captured image into the virtually cleansed intestinal tract image.

4. The method according to claim 3, wherein
the learning model is a model obtained by using, as teaching data, a residue-containing image that is an image of a large intestine part including a residue and a residue-removed image that is an image of the large intestine part after removing the residue to obtain an image processing parameter for obtaining the residue-removed image from the residue-containing image.

5. A program causing a computer to execute a method for acquiring a virtually cleansed intestinal tract image, the method including:

inputting a captured image that is an image obtained by capturing a portion of a large intestine of a patient; and
obtaining a virtually cleansed intestinal tract image that is an image of the portion of the large intestine of the patient after removing a part that corresponds to residue in the large intestine included in the captured image.

6. A non-transitory computer-readable storage medium storing the program according to claim 5.

7. A method for detecting a polyp from a captured image, the method comprising:

obtaining a large intestine image that is a partial image obtained by extracting only a large intestine part included in a captured image that is an image obtained by capturing a portion of a large intestine of a patient from the captured image;
obtaining a polyp image that is an image obtained by extracting a polyp part included in the large intestine image; and
removing a false positive included in the polyp image.

8. The method according to claim 7, wherein
obtaining a large intestine image uses a learning model for extracting the large intestine to directly convert the captured image into the large intestine image.

9. The method according to claim 8, wherein
the learning model for extracting the large intestine is a model obtained by using, as teaching data, a large intestine ground truth region image that is an image obtained by removing other organ parts than the large intestine from a certain captured image and extracting only the large intestine part.

10. The method according to claim 7, wherein
obtaining a polyp image uses a learning model to directly convert the large intestine image into the polyp image.

11. The method according to claim 10, wherein
the learning model is a model obtained by using, as teaching data, a certain large intestine image and a polyp ground truth region image that is an image obtained by extracting only a region of a polyp from the certain large intestine image.

12. A program causing a computer to execute a method for detecting a polyp from a captured image, the method including:

obtaining a large intestine image that is a partial image obtained by extracting only a large intestine part included in a captured image that is an image obtained by capturing a portion of a large intestine of a patient from the captured image;
obtaining a polyp image that is an image obtained by extracting a polyp part included in the large intestine image; and
removing a false positive included in the polyp image.

13. A non-transitory computer-readable storage medium storing the program according to claim 12.

[Fig.1]

```
┌─────────────────────────────────────┐
│  Learning model construction step   │ ───── S001
└─────────────────────────────────────┘
                    │
┌─────────────────────────────────────┐
│          Image input step           │ ───── S101
└─────────────────────────────────────┘
                    │
┌─────────────────────────────────────┐
│       Virtual intestinal tract      │ ───── S102
│           cleansing step            │
└─────────────────────────────────────┘
                    │
┌─────────────────────────────────────┐
│     Three-dimensional endoscopy-    │ ───── S103
│    like image generation step       │
└─────────────────────────────────────┘
```

[Fig.2]

[Fig.3]
Flowchart of the virtual large intestine cleansing step

[Fig.4]

(a)                        (b)                        (c)

(d)                                    (e)

[Fig.5]

Image input step(S101)

↓

Large intestine extraction step(S202) ← Large intestine extraction learning model construction step(S302)

↓

The polyp extraction step (S203) ← Polyp extraction learning model construction step(S303)

↓

False positive removal step(S204) ← False positive removal learning model construction Step (S304)

[Fig.6]

[Fig.7]

[Fig.8]

[Fig.9]

Image of polyp

Model training step

Image of polyp → Output true polyp label

Training model

False positive removal step

Image of polyp

Input to trained model

Output determining whether polyp image contains a true polyp

[Fig.10]

(a)

(b)

[Fig.11]

(a)　　　　　　　　　　　　(b)

[Fig.12]

(a)　　　　　　　　　(b)　　　　　　　　　(c)

[Fig.13]

(a)　　　　　　　　　　　　　　　　　　(b)

## INTERNATIONAL SEARCH REPORT

| International application No. |
| --- |
| **PCT/JP2024/004959** |

| A. | CLASSIFICATION OF SUBJECT MATTER |
| --- | --- |

*A61B 6/03*(2006.01)i; *A61B 1/00*(2006.01)i
FI:   A61B6/03 560G; A61B1/00 V; A61B6/03 560T

According to International Patent Classification (IPC) or to both national classification and IPC

| B. | FIELDS SEARCHED |
| --- | --- |

Minimum documentation searched (classification system followed by classification symbols)

A61B6/00-6/58; G06T1/00-19/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | 橘理恵ほか, CTコロノグラフィにおけるGANを用いたクレンジング画像の取得, INNERVISION, 2019, 34, 7, 39-42, <URL: https://mol.medicalonline.jp/library/archive/search?jo=ab0inerc&ye=2019&vo=34&issue=7>, (TACHIBANA, Rie et al.), non-official translation () "GANを用いたクレンジング画像の生成", fig. 5, 6 | 1-6 |
| X | NAPPI et al. Fully Automated Three-Dimensional Detection of Polyps in Fecal-Tagging CT Colonography. Academic Radiology. 2007, 14, 3, 287-300, <URL: https://www.sciencedirect.com/science/article/pii/S1076633206006738> fig. 1 | 7, 12-13 |
| Y | fig. 1 | 8-11 |
| Y | WO 2022/244002 A1 (RAMOT AT TEL-AVIV UNIVERSITY LTD.) 24 November 2022 (2022-11-24) paragraphs [0046], [0067]-[0078], fig. 1 | 8-11 |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **28 March 2024** | **09 April 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 767 946 A1

## INTERNATIONAL SEARCH REPORT

| International application No. |
| --- |
| **PCT/JP2024/004959** |

### C.   DOCUMENTS CONSIDERED TO BE RELEVANT

| Category* | Citation of document, with indication, where appropriate, of the relevant passages | Relevant to claim No. |
| --- | --- | --- |
| A | JP 2008-510565 A (SIEMENS MEDICAL SOLUTIONS USA, INC.) 10 April 2008 (2008-04-10) | 1-13 |
| A | US 2010/0021026 A1 (COLLINS et al.) 28 January 2010 (2010-01-28) | 1-13 |
| A | US 2011/0206250 A1 (MCGINNIS et al.) 25 August 2011 (2011-08-25) | 1-13 |
| A | WO 2022/117470 A1 (KONINKLIJKE PHILIPS N.V.) 09 June 2022 (2022-06-09) | 1-13 |
| A | TACHIBANA et al. Electronic cleansing in CT colonography using a generative adversarial network. Proc. of SPIE. 2019, 10954, 1095419-1-1095419-6, <URL: https://doi.org/10.1117/12.2512466> | 1-13 |
| A | NAPPI et al. Feasibility of the automated detection of colorectal polyps in photon-counting CT colonography. Proc. of SPIE. 10 April 2023, 12469, 124690T-1 to 124690T-5, <URL: https://doi.org/10.1117/12.2654292> | 1-13 |

Form PCT/ISA/210 (second sheet) (July 2022)

23

## INTERNATIONAL SEARCH REPORT

International application No.

**PCT/JP2024/004959**

---

**Box No. III    Observations where unity of invention is lacking (Continuation of item 3 of first sheet)**

This International Searching Authority found multiple inventions in this international application, as follows:

(Invention 1) Claims 1-6
    Claims 1-4 lack novelty in light of document 1 cited in the international search report, and thus do not have a special technical feature.
    Claims 1-4 are classified as invention 1 and also claims 5-6 are substantially identical to or similarly closely related to claim 1 classified as invention 1, and are thus classified as invention 1.

(Invention 2) Claims 7-13
    Claims 7-13 cannot be said to have a technical feature identical or corresponding to that of claim 1 classified as invention 1, do not depend from claim 1, and are not substantially identical to or similarly closely related to any of the claims classified as invention 1.
    Therefore, claims 7-13 cannot be classified as invention 1.
    In addition, claims 7-11 are classified as invention 2, and claims 12-13 are substantially identical to or similarly closely related to claim 7 classified as invention 2 and are thus classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

---

Form PCT/ISA/210 (continuation of first sheet) (July 2022)

### INTERNATIONAL SEARCH REPORT
**Information on patent family members**

International application No.

**PCT/JP2024/004959**

| Patent document cited in search report | | | Publication date (day/month/year) | Patent family member(s) | | | Publication date (day/month/year) |
|---|---|---|---|---|---|---|---|
| WO | 2022/244002 | A1 | 24 November 2022 | (Family: none) | | | |
| JP | 2008-510565 | A | 10 April 2008 | US | 2006/0047227 | A1 | |
| | | | | WO | 2006/023152 | A1 | |
| | | | | CA | 2578334 | A | |
| | | | | CN | 101048797 | A | |
| US | 2010/0021026 | A1 | 28 January 2010 | (Family: none) | | | |
| US | 2011/0206250 | A1 | 25 August 2011 | (Family: none) | | | |
| WO | 2022/117470 | A1 | 09 June 2022 | EP | 4009271 | A1 | |
| | | | | CN | 116547697 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7304594 B